# EUROPÄISCHE PATENTSCHRIFT

(11) **EP 1 187 563 B1**
(45) Veröffentlichungstag und Bekanntmachung des Hinweises auf die Patenterteilung: **30.11.2005**
(21) Anmeldenummer: 00941984.7
(22) Anmeldetag: 25.05.2000
(51) Int. Cl.: A61B 17/22, A61N 7/00

(54) **MEDIZINISCHES INSTRUMENT ZUR BEHANDLUNG VON BIOLOGISCHEM GEWEBE SOWIE VERFAHREN ZUM ÜBERTRAGEN VON DRUCKWELLEN**
MEDICAL INSTRUMENT FOR TREATING BIOLOGICAL TISSUE AND METHOD FOR TRANSMITTING PRESSURE WAVES
INSTRUMENT MEDICAL DE TRAITEMENT DE TISSU BIOLOGIQUE ET PROCEDE DE TRANSMISSION D'ONDES DE PRESSION

(30) Priorität: 24.06.1999 DE 19929112
(43) Veröffentlichungstag der Anmeldung: 20.03.2002
(73) Patentinhaber: Ferton Holding SA, 2800 Delémont (CH)
(72) Erfinder: MENNE, Andreas, D-88709 Meersburg (DE); JERGER, Thomas, D-71065 Sindelfingen (DE)
(74) Vertreter: Dallmeyer, Georg
(86) Internationale Anmeldenummer: PCT/EP2000/004771
(87) Internationale Veröffentlichungsnummer: WO 2001/000094

(56) Entgegenhaltungen:
- WO-A-94/17771
- WO-A-96/25888
- WO-A-96/28213
- WO-A-98/07470
- WO-A-98/32379
- DE-A- 19 725 477
- US-A- 4 603 701
- US-A- 5 273 027
- US-A- 5 494 038

## Beschreibung

Die Erfindung betrifft ein medizinisches Instrument zur Behandlung von biologischem Gewebe mittels unfokussierter Druckwellen.

Derartige Instrumente dienen dazu, mittels Druck- oder Stoßwellen den Heilungsprozeß bei Knochenbrüchen und Knochendefekten aber auch bei der Parodontose zu beschleunigen oder überhaupt erst in Gang zu bringen. Ein weiteres Einsatzgebiet ist die Behandlung von Schmerzen bei Sehnenansatzerkrankungen.

Bei bislang für solche Behandlung verwendeten bekannten, extrakorporalen Druckwellengeräte wird im Brennpunkt eines akustischen Reflektors, z.B. mittels einer Funkenentladung, wie in der Deutsche Offenlegungsschrift DE 23 51 247 A angegeben, eine impulsförmige Druck- oder Stoßwelle erzeugt, die dann durch den Reflektor auf das zu beschallende Objekt fokussiert wird. Es wird vermutet, daß mit Hilfe der Druckwellen Mikroschädigungen im biologischen Gewebe erzeugt werden, die den Körper zu Regenerationsmaßnahmen veranlassen.

Solche bekannten Druckwellengeräte besitzen einen eng lokalisierten Fokusbereich, in dem allein eine therapeutische Wirkung beobachtet wird. Dies ist darin begründet, daß diese Druckwellengeräte - sogenannte extrakorporale Lithotriptoren - für die Zertrümmerung von Körpersteinen entwickelt wurden. Bei dieser Anwendung wird die gesamte Energie in einem kleinen Areal, in diesem Fall auf den zu zerstörende Körperstein, gebündelt. Für ein befriedigendes Behandlungsergebnis bei Knochenbrüchen und in der Schmerztherapie hingegen, muß meist ein größeres Areal gleichmäßig beschallt werden. Dies verlangt einen aufwendigen Bewegungsmechanismus und ist zudem durch das wiederholte Aufsuchen der Behandlungsposition sehr zeitintensiv.

Ein weiterer Nachteil der extrakorporalen Druckwellenquellen ist die Unsichtbarkeit des Fokusbereiches für den Anwender. Der Brennpunkt liegt außerhalb des eigentlichen Gerätes. Für eine Behandlung benötigt der Arzt daher ein Ortungssystem (Ultraschall oder/und Röntgen) um Fokus und Behandlungsort zur Deckung zu bringen.

In der Deutschen Offenlegungsschrift DE 197 25 477 A1 ist ein Gerät beschrieben, bei dem mit einem Übertragungselement unfokussierte, mechanische erzeugte Druckwellen in biologisches Gewebe eingekoppelt werden. Dabei muß der Anwender das stumpfe Übertragungselement auf den Behandlungsort richten. Dieses sehr einfach aufgebaute Instrument hat allerdings zur Folge, daß die Bewegung des Übertragungselementes - auch wenn sie sich nur auf eine Längenänderung durch den Einfluß einer Druckwelle beschränkt - nicht vermeidbar ist. Für Anwendungen, bei denen ein solches Gerät auf die Hautoberfläche angesetzt wird, mag die Belastung durch die Auslenkung des Übertragungselementes, wie Rötungen und leichte Schwellungen, vertretbar sein. Bei der Behandlung einer Parodontose hingegen, müßte ein solches Instrument auf das Zahnfleisch aufgesetzt werden, welches dieser Belastung nicht standhält.

Das Dokument WO-A-94/17 771 offenbart ein medizinisches Instrument mit einer Druckwellenerzeugungseinrichtung und einer Druckkammer, die Druckwellen überträgt und in biologisches Gewebe einkoppelt, wobei die Druckkammer aus einem langgestreckten, flüssigkeitsgefüllten Kanal besteht, und wobei ein linear hin- und herbewegbares Schlagteil in die Flüssigkeit der Durckkammer Druckwellen einkoppelt.

Der Erfindung liegt demzufolge die Aufgabe zugrunde, ein medizinisches Instrument zur Übertragung von Druckwellen so auszubilden, daß es auf eine einfache und kostengünstige Weise eine gleichmäßige Energieverteilung der Druckwelle auf einen großflächigen Wirkungsbereich ermöglicht, ohne bei der Übertragung der Druckwelle von dem Instrument auf das biologische Gewebe dasselbe zu schädigen.

Zur Lösung dieser Aufgabe dienen die Merkmale des Anspruchs 1.

Die Erfindung sieht vor, daß die Übertragungseinrichtung eine flüssigkeitsgefüllte Druckkammer aufweist, deren Flüssigkeit eingekoppelte. Druckwellen auf eine an dem ausgangsseitigen Ende der Druckkammer angeordnete Membran überträgt, die die von der Flüssigkeit übertragenen Druckwellen unfokussiert in das biologische Gewebe einkoppelt.

Da die Impedanz der Flüssigkeit weitgehend der Impedanz des biologischen Gewebes entspricht, in die die Druckwelle eingekoppelt werden soll, entstehen bei der Übertragung der Druckwelle von der Flüssigkeit über die Membran auf das biologische Gewebe keine Übertragungsverluste. Insbesondere kann eine gleichmäßige Energieverteilung der Druckwelle auf einen großflächigen Wirkungsbereich ermöglicht werden, ohne das biologische Gewebe bei der Übertragung der Druckwelle zu schädigen.

Als Einkoppeleinrichtung wird eine dünne Membran verwendet. Diese verschließt einerseits die Druckkammer an ihrem ausgangsseitigen Ende und ist andererseits bei der Einkopplung der Druckwellen aus der Flüssigkeit in das biologische Gewebe nicht hinderlich. Auch wird die Eintrittsfläche des biologischen Gewebes bei der Weiterleitung der Druckwelle durch die Membran mechanisch nicht belastet, so daß der Einsatz des Instrumentes für alle Arten von biologischem Gewebe möglich.ist. Als Membran können impedanzangepaßte Kunststoffolien verwendet werden.

Die Druckkammer besteht aus einem langgestreckten Kanal. Die Kanalform hat den Vorteil, daß die von der Druckwellenerzeugungseinrichtung eingekoppelte Druckwelle nicht seitlich auswandern kann und auf diese Weise über eine längere Distanz als ebene Welle ohne größere Verluste transportiert werden kann. Darüber hinaus kommt es in diesem rohrförmigen Kanal infolge der Lauflänge und den nicht linearen Fortpflanzungseigenschaften zu einem Aufsteilen der Druckwelle, so daß der langgestreckte Kanal in der Art eines Stoßwellenrohres arbeitet. Durch das Aufsteilen der Druckwelle ändern sich die physikalischen Zustandsgrößen der Druckwelle innerhalb einer kürzeren Zeitspanne und die zerstörenden Eigenschaften der Druckwelle nehmen zu, ein Effekt, der für das Setzen der Mikroschäden von Vorteil ist.

Das Verhältnis der Länge der Druckkammer zu ihrem Durchmesser liegt vorzugsweise im Bereich zwischen 2 und 10.

Die Druckwellenerzeugungseinrichtung kann innerhalb der Druckkammer angeordnet sein. Die Druckwellenerzeugungseinrichtung generiert eine nicht gerichtete Druckwelle, die sich kugelförmig ausbreitet und deren Energie demzufolge mit der Entfernung von ihrem Ursprungsort rasch abnimmt. Liegen Entstehungs- und Anwendungsort nicht nahe beieinander, so erreicht nur ein geringer Teil der eingesetzten Energie seinen Bestimmungsort. Um diese Verluste zu beschränken, kann die mit Flüssigkeit gefüllte Druckkammer so ausgebildet sein, daß die Druckwellenerzeugungseinrichtung innerhalb des rohrförmigen Kanals in der Nähe seines eingangsseitigen Endes angeordnet ist.

Bei der Erfindung ist vorgesehen, daß die Druckwellenerzeugungseinrichtung die Druckwellen mechanisch in die Druckkammer einkoppelt und daß ein linear geführtes, in Axialrichtung elastisch gelagertes Übertragungselement mit einer Austrittsgrenzfläche an die Flüssigkeit der Druckkammer angekoppelt ist und mechanisch induzierte Druckwellen auf die Flüssigkeit überträgt.

Das Übertragungselement wird von einem linear hin- und herbewegbaren Schlagteil beaufschlagt, das infolge des Kraftstoßes mechanisch eine Druckwelle induziert, die sich bis zur Austrittsgrenzfläche der Membran bzw. des Übertragungselementes fortpflanzt.

Dabei ist das Schlagteil koaxial zu dem Übertragungselement geführt.

Die Druckwellenerzeugungseinrichtung erzeugt die eingekoppelten Druckwellen periodisch. Eine größere Anzahl von hintereinanderfolgenden einzelnen impulsförmigen Druckwellen mit schwächerer Energie zeigt einen besseren Heilungserfolg in dem biologischen Gewebe als ein einzelner starker Druckimpuls. Die Druckwellenerzeugungseinrichtung kann daher mit einer Wiederholfrequenz einzelner Druckimpulse zwischen 1 und 20 Hz arbeiten, wobei zur Behandlung des biologischen Gewebes etwa 1000 bis.2000 Druckimpulse erforderlich sind.

Bei einem mechanischen Betrieb der Drückwellenerzeugungseinrichtung sind die Antriebsmittel für einen kontinuierlichen Betrieb vorzugsweise so ausgestaltet, daß eine periodische Bewegung des Antriebs möglich ist. Wird beispielsweise ein pneumatisches Schlagteil verwendet, das durch seinen Aufschlag auf ein Übertragungselement in diesem eine Druckwelle erzeugt, so können die Luftströme so ausgelegt sein, daß das Schlagteil eine kontinuierliche Hin- und Herbewegung ausführt und periodisch das übertragungselement trifft.

Elektrisch betriebene Druckwellenerzeugungseinrichtungen werden in der Regel von einer Kondensatorbatterie versorgt. Hier muß bei einem periodischen Betrieb die Stromzuführung für das Laden der Batterien ausreichend hoch sein, damit die vorgegebene Anzahl von Druckimpulsen mit der nötigen Wiederholfrequenz abgegeben werden kann.

Bei weiteren Ausführungsbeispielen der Erfindung ist vorgesehen, daß der Querschnitt des Kanals in Richtung auf die äußere Membran konisch erweitert oder verjüngt ist. Eine sich in Richtung der Membran verjüngende Druckkammer verstärkt die Druckwelle durch die Verkleinerung ihres Querschnitts. Eine Erweiterung der Druckkammer schwächt demgegenüber die Druckwelle ab, beschallt aber eine größere Behandlungsfläche.

Zwischen der äußeren Membran und dem biologischen Gewebe kann ein impedanzanpassendes Medium angeordnet sein, das das Einkoppeln der Druckwellen in das biologische Gewebe verbessert. Liegt die Membran nicht absolut eben und ohne Lufteinschlüsse auf dem biologischen Gewebe auf, so wird ein Teil des Druckimpulses an dieser akustischen Unstetigkeit reflektiert und der Anteil der einkoppelbaren Druckwellen gemindert. Ein geeignetes pastenförmiges Impedanzanpassungsmedium ist beispielsweise ein Ultraschallgel oder andere pastenförmige Massen mit einer ähnlichen Impedanz wie das biologische Gewebe, wie beispielsweise Vaseline.

Im folgenden werden unter Bezugnahme auf die Zeichnungen Ausführungsbeispiele der Erfindung näher erläutert. Es zeigen:
- Fig. 1: ein erstes Ausführungsbeispiel mit einer mechanischen Druckwellenerzeugungseinrichtung,
- Fig. 2: ein Gerät, das nicht Teil der Erfindung ist, mit einer elektrohydraulischen Sonde als Druckwellenerzeugungseinrichtung,
- Fig. 3: ein Gerät, das nicht Teil der Erfindung ist, mit einer piezoelektrischen Druckwellenerzeugungseinrichtung,
- Fig. 4: ein Gerät, das nicht Teil der Erfindung ist, mit einer elektromagnetischen Druckwellenerzeugungseinrichtung,
- Fig. 5: eine Übertragungseinrichtung mit einer konisch sich verjüngenden Druckkammer, und
- Fig. 6: eine Übertragungseinrichtung mit einer sich konisch erweiternden Druckkammer.

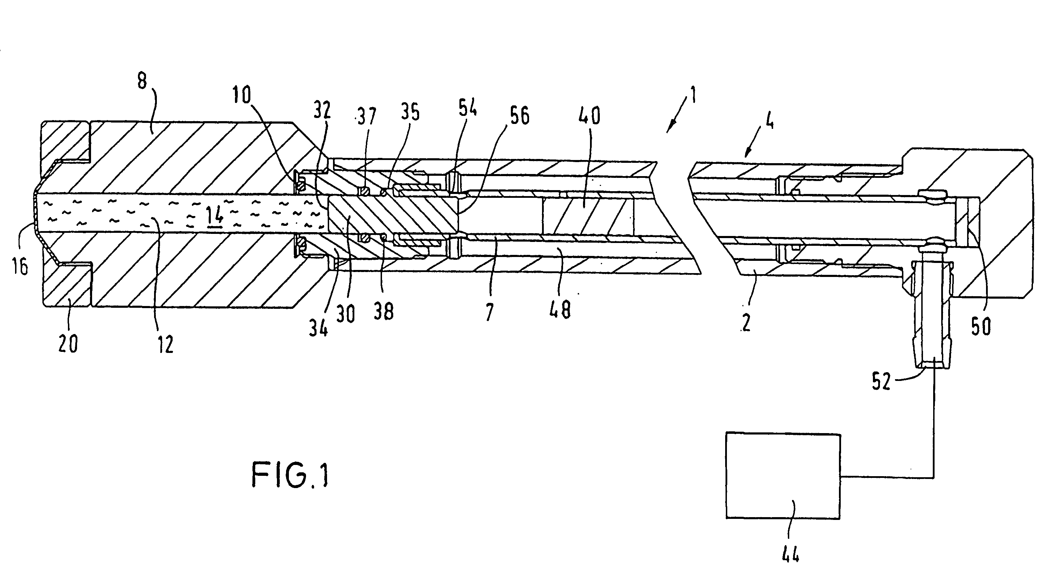

Das in Fig. 1 gezeigte Handstück 1 besteht aus einem Gehäuse 2 einer mechanischen Druckwellenerzeugungseinrichtung 4 mit einem Innenzylinder 7, in dem ein Schlagteil 40 mit Hilfe pneumatischer Antriebsmittel 44 in Verbindung mit einer Staudruckkammer 48, die den Innenzylinder 7 koaxial ringförmig umgibt, zwischen zwei Endpositionen hin- und herbewegt wird. An dem distalen Ende des Gehäuses 2 ist eine Übertragungseinrichtung 8 für Druckwellen aufgeschraubt.

Alternativ ist es auch möglich, das Schlagteil 40 hydraulisch, mechanisch, elektromagnetisch oder durch andere Antriebsmittel zu bewegen. Je nach Antriebsart kann die Länge des Beschleunigungsweges ausgewählt werden. Bei einem pneumatisch betriebenen Schlagteil 40 und einem pneumatischen Druck von ca. 0,3 MPa (3 bar) beträgt dieser etwa 50 bis 200 mm. In der proximalen Endposition des Schlagteils 40 am Ende des Innenzylinders 7 ist ein Magnethalter 50 angeordnet, der das metallische Schlagteil 40 in seiner proximalen Endposition festhalten kann, bis erneut ein über den Anschluß 52 aufgebrachter pneumatischer Druck das Schlagteil 40 in Richtung auf das distale Ende des Innenzylinders 7 beschleunigt. Die sich in Bewegungsrichtung des Schlagteils 40 befindliche Luft vor dem Schlagteil 40 wird über an dem distalen Ende des Innenzylinders 7 befindliche Ringschlitze 54 in die Staudruckkammer 48 geleitet. Durch die Beschleunigung des Schlagteils 40 trifft dieses mit hoher Endgeschwindigkeit von beispielsweise 10 - 25 m/s auf eine distal von dem Innenzylinder 7 angeordnete Eintrittsgrenzfläche 56 eines Übertragungselementes 30. Das Übertragungselement 30 besteht aus einer im wesentlichen zylindrischen metallischen Sonde mit einer plan oder leicht nach innen oder außen gewölbten Austrittsgrenzfläche 32. Das Übertragungselement 30 wird in einem hohlzylindrischen Aufnahmeteil 34 gleitend geführt. Ein Ringbund 35 am Übertragungselement 30 dient als Anschlagteil gegen das Aufnahmeteil 34, wobei zwischen Ringbund 35 und dem Aufnahmeteil 34 ein Feder-/Dämpfungselement 38 angeordnet ist, das das Übertragungselement 30 von dem Aufnahmeteil 34 entkoppelt und auch dafür sorgt, daß das Übertragungselement 30 nach dem Schlagvorgang wieder in seine Ausgangsposition am distalen Ende des Innenzylinders 7 zurückkehrt. Ein das Übertragungselement 30 gleitend aufnehmender O-Ring 37 dichtet die Druckwellenerzeugungseinrichtung 4 gegen die Übertragungseinrichtung 8 für die Druckwellen ab.

Die Austrittsgrenzfläche 32 des Übertragungselementes 30 ist in direktem Kontakt mit einer mit Flüssigkeit gefüllten Druckkammer 12 der Übertragungseinrichtung 8. Als Flüssigkeit dient bevorzugt eine den akustischen Eigenschaften von biologischem Gewebe ähnliche Substanz, wie z.B. Wasser. Die Druckkammer 12 kann eine längliche zylindrische Form aufweisen, um die Druckwelle aufzusteilen, kann aber auch sehr kurz gehalten sein, wenn die Druckwellenerzeugungseinrichtung 4 Druckwellen ausreichender Intensität erzeugt. Die Druckkammer 12 wird an dem distalen Ende von einer Membran 16 verschlossen. Eine Spannvorrichtung 20 spannt die Membran 16 über das distale Ende der Druckkammer 12 und schließt deren ausgangsseitiges Ende dicht gegen die Umgebung ab, so daß die Flüssigkeit 14 nicht aus der Druckkammer 3 heraustreten kann.

Schlägt das Schlagteil 40 auf die Eingangsgrenzfläche 56 des Übertragungselementes 30 auf, wird eine Druckwelle in dem Übertragungselement 30 erzeugt, die sich bis zu der Austrittsgrenzfläche 32 des Übertragungselementes 30 fortpflanzt und dann in die Flüssigkeit 14 innerhalb der Druckkammer 12 eingekoppelt wird. Die Druckwelle läuft innerhalb der Druckkammer 12 in Richtung auf die distale Membran 16, steilt sich dabei durch die nicht linearen Wellenausbreitungsbedingungen auf und tritt durch die Membran 16 hindurch in das die Membran 16 kontaktierende biologische Gewebe ein.

Nach Beendigung des Schlagvorgangs bewegt das Feder-/Dämpfungselement 38 das Übertragungselement 30 wieder in seine Ausgangsposition zurück. Das Schlagteil 40 wird durch den in der Staudruckkammer 48 aufgebauten Überdruck und das Zurückströmen der Luft aus der Staudruckkammer 48 durch die Ringschlitze 54 hindurch in seine Ruheposition am proximalen Ende des Innenzylinders 7 zurückgeführt und von dem Magnethalter 50 gehalten. Das Instrument ist nun wieder zu einem erneuten Schlagvorgang bereit.

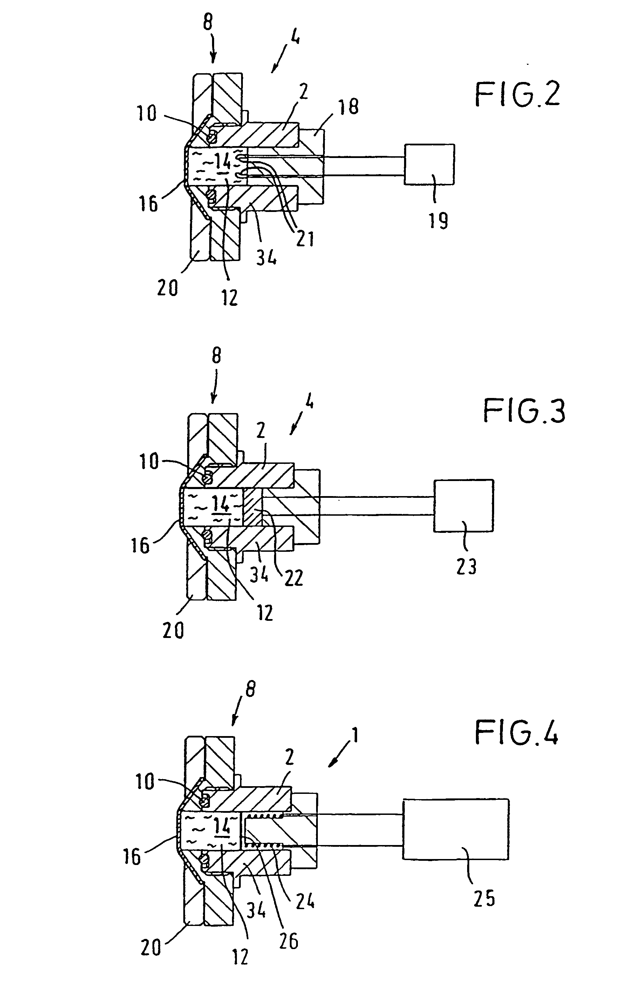

Fig. 2 zeigt ein Gerät mit einer Funkenentladungsstrecke zur Erzeugung von Druckwellen. Eine elektrische Schaltung 19 versorgt die beiden Elektroden 21 einer elektrohydraulischen Sonde 18 mit einem kurzen Spannungsimpuls. Ist die umgebende Flüssigkeit 14 in der Druckkammer 12 elektrisch leitend, so kommt es zu einem Funkenüberschlag zwischen den Elektroden 21. Durch die damit einhergehende plötzliche Plasmabildung entsteht eine Druckwelle, die sich in der Druckkammer 12 ausbreitet und über die Membran 16 in das mit der Membran 16 in Kontakt stehende biologische Gewebe eingekoppelt wird.

Ein weiteres Gerät mit einer Druckwellenerzeugungseinrichtung 3 ist in Fig. 3 dargestellt. Eine elektrische Schaltung 23 liefert einen Spannungsimpuls an ein in der Druckkammer 12 angeordnetes piezoelektrisches Element 22. Infolge des Spannungsimpulses dehnt sich das piezoelektrische Element 22 aus und generiert in der umgebenden Flüssigkeit 14 eine Druckwelle.

Fig. 4 zeigt ein weiteres Gerät mit einer Druckwellenerzeugungseinrichtung 4 mit einer Spulenanordnung 24, die von einer elektrischen Schaltung 25 mit Strom versorgt wird. Distal vor der Spulenanordnung 24 ist eine Erregermembran 26 angeordnet. Die Spulenanordnung 24 induziert in der Erregermembran 26 einen Wirbelstrom, der wiederum ein Magnetfeld aufbaut. Durch die abstoßenden Kräfte zwischen der Spulenanordnung 24 und der Erregermembran 26 wird letztere bei einem kurzen Stromimpuls aus der elektrischen Schaltung 25 ruckartig von der elektrischen Spulenanordnung 24 wegbewegt, wodurch eine Druckwelle in die Druckkammer 12 eingekoppelt wird.

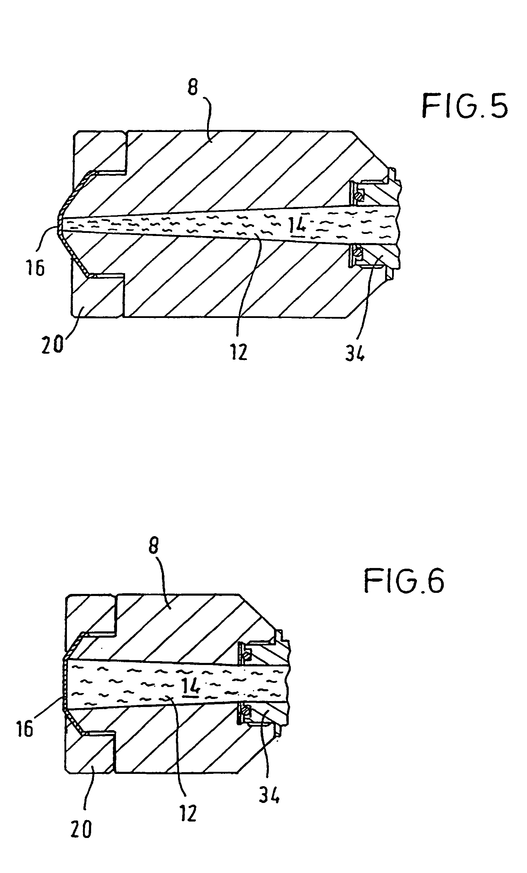

Die Übertragungseinrichtungen 8 der unterschiedlichen Ausführungsbeispiele unterscheiden sich durch die Länge und die Form der Druckkammer 12. Fig. 5 zeigt eine Übertragungseinrichtung 8, bei der sich die Druckkammer 12 in distaler Richtung konisch verjüngt. Eine von einer Druckwellenerzeugungseinrichtung 4 erzeugte Druckwelle wird infolge der Verkleinerung des wirksamen Querschnitts der Druckkammer 12 verstärkt. Erweitert sich die Druckkammer 12 wie in Fig. 6 dargestellt, so wird die Druckwelle abgeschwächt, beschallt aber eine größere Fläche. Ist ein Aufsteilen der Druckwelle nicht erforderlich oder nicht erwünscht, so kann die Druckkammer 12 kurz gehalten sein, wie bei den Ausführungsbeispielen der Fign. 2 bis 4.

Die Übertragungseinrichtung 8 ist als Schraubkopf gestaltet und kann auf die Druckwellenerzeugungseinrichtung 4 aufgeschraubt werden. Eine Dichtung 10 dichtet die Übertragungseinrichtung 8 gegen das Aufnahmeteil 34 der Druckwellenübertragungseinrichtung 4 ab. Die Druckkammer 12 kann sich dabei bis in das Aufnahmeteil 34 hinein erstrecken.

## Patentansprüche

1. Medizinisches Instrument zur Behandlung von biologischem Gewebe, mit einem Gehäuse (2), mit einer Druckwellenerzeugungseinrichtung (4) und mit einer Übertragungseinrichtung (8) zum Einkoppeln der unfokussierten Druckwellen in den Körper von Lebewesen, wobei
die Übertragungseinrichtung (8) eine Druckkammer (12) mit einem eingangsseitigen und einem ausgangsseitigen Ende aufweist, die eine Flüssigkeit (14) enthält, in die Druckwellen aus der Druckwellenerzeugungseinrichtung (4) einkoppelbar und an dem ausgangsseitigen Ende der Druckkammer (12) auf das biologische Gewebe übertragbar sind, wobei die von der Flüssigkeit übertragenen Druckwellen unfokussiert in das biologische Gewebe einkoppelbar sind,
wobei am ausgangsseitigen Ende der Druckkammer (12) eine Membran (16) angeordnet ist,
wobei die Druckkammer (12) aus einem Kanal besteht, in dem ein in Axialrichtung linear geführtes Übertragungselement (30) elastisch gelagert ist, wobei das Übertragungselement (30) mit einer Austrittsgrenzfläche (32) an die Flüssigkeit (14) der Druckkammer (12) angekoppelt ist, und
wobei das Übertragungselement (30) von einem linear hin- und herbewegbaren Schlagteil (40) beaufschlagt ist, das infolge des Kraftstoßes mechanisch eine Druckwelle induziert.

2. Instrument nach Anspruch 1, wobei das Verhältnis zwischen Länge und Durchmesser der kanalförmigen Druckkammer (12) im Bereich zwischen 2 und 10 liegt.

3. Instrument nach Anspruch 1 oder 2, wobei
das Schlagteil (40) koaxial zu dem Übertragungselement (30) geführt ist.

4. Instrument nach einem der Ansprüche 1 bis 3, wobei die Druckwellenerzeugungseinrichtung (4) die Druckwellen periodisch erzeugt.

5. Instrument nach einem der Ansprüche 1 bis 4, wobei
der Querschnitt der Druckkammer (12) in distaler Richtung auf die Membran (16) konisch erweitert ist.

6. Instrument nach einem der Ansprüche 1 bis 4, wobei
der Querschnitt der Druckkammer (12) in distaler Richtung auf die Membran (16) konisch verengt ist.

7. Instrument nach einem der Ansprüche 1 bis 6, wobei die Membran (16) auswechselbar an
der Übertragungseinrichtung (8) befestigt ist.

8. Instrument nach einem der Ansprüche 1 bis 7, wobei
eine Spanneinrichtung (20) die Membran (16) unter Vorspannung hält.

9. Instrument nach einem der Ansprüche 1 bis 8, wobei
die Membran (16) aus einer fluiddichten impedanzangepaßten Kunststoffolie besteht.

10. Instrument nach einem der Ansprüche 1 bis 9, wobei
die in der Druckkammer (12) enthaltene Flüssigkeit (14) salzhaltig ist.

11. Instrument nach einem der Ansprüche 1 bis 10, wobei
zwischen der Membran (16) und dem biologischen Gewebe ein impedanzanpassendes Medium angeordnet ist, das das Einkoppeln der Druckwellen in das biologische Gewebe verbessert.

## Claims

1. A medical instrument for treating biological tissue, the instrument comprising a housing (2), a pressure wave generation means (4) and a transmission means (8) for coupling the unfocused pressure waves into the body of living beings, wherein
the transmission means (8) comprises a pressure chamber (12) having an inlet end and an outlet end, the pressure chamber (12) containing a liquid (14) into which the pressure waves from the pressure wave generation means (4) can be coupled, which pressure waves can be transmitted from the outlet end of the pressure chamber (12) to the biological tissue, wherein the pressure waves transmitted by the liquid can be unfocusedly coupled into the biological tissue,
wherein at the outlet end of the pressure chamber (12) a membrane (16) is arranged,
wherein the pressure chamber (12) comprises a duct in which a transmission element (30) linearly guided in axial direction is elastically supported, wherein the transmission element (30) is coupled at an exit interface (32) to the liquid (14) in the pressure chamber (12), and
wherein the transmission element (30) is energized by a linearly reciprocating beater part (40) which, as a result of the impulse, mechanically induces a pressure wave.

2. The instrument according to claim 1, wherein the ratio between length and diameter of the duct-shaped pressure chamber (12) ranges between 2 and 10.

3. The instrument according to claim 1 or 2, wherein the beater part (40) is guided coaxially to the transmission element (30).

4. The instrument according to one of claims 1 to 3, wherein the pressure wave generation means (4) periodically generates the pressure waves.

5. The instrument according to one of claims 1 to 4, wherein the crosssection of the pressure chamber (12) is flared in distal direction towards the membrane (16).

6. The instrument according to one of claims 1 to 4, wherein the crosssection of the pressure chamber (12) tapers in distal direction towards the membrane (16).

7. The instrument according to one of claims 1 to 6, wherein the membrane (16) is exchangeably fastened to the transmission means (8).

8. The instrument according to one of claims 1 to 7, wherein a tensioning means (20) keeps the membrane (16) under pretension.

9. The instrument according to one of claims 1 to 8, wherein the membrane (16) comprises a fluid-tight and impedance-matched plastic film.

10. The instrument according to one of claims 1 to 9, wherein the liquid (14) contained in the pressure chamber (12) is saliferous.

11. The instrument according to one of claims 1 to 10, wherein between the membrane (16) and the biological tissue an impedance-matched medium is arranged which improves coupling of the pressure waves into the biological tissue.

## Revendications

1. Instrument à usage médical pour le traitement de tissu biologique, lequel instrument comporte un boîtier (2), un dispositif de génération d'ondes de pression (4) et un dispositif de transmission (8) pour l'injection des ondes de pression non focalisées dans le corps d'êtres vivants,
le dispositif de transmission (8) comportant une chambre de pression (12) qui présente une extrémité d'entrée et une extrémité de sortie et qui contient un liquide (14) dans lequel des ondes de pression provenant du dispositif de génération d'ondes de pression (4) peuvent être injectées et transmises au tissu biologique à l'extrémité de sortie de la chambre de pression (12), les ondes de pression transmises par le liquide pouvant être injectées de façon non focalisée dans le tissu,
une membrane (16) étant placée à l'extrémité de sortie de la chambre de pression (12),
la chambre de pression (12) se composant d'un canal dans lequel un élément de transmission (30), guidé linéairement dans une direction axiale, est monté élastiquement,
l'élément de transmission (30) étant couplé par une surface limite de sortie (32) au liquide (14) de la chambre de pression (12), et
l'élément de transmission (30) étant sollicité par une pièce de percussion (40) qui est mobile linéairement suivant un mouvement alternatif et qui induit mécaniquement une onde de pression à la suite d'une impulsion.

2. Instrument selon la revendication 1, dans lequel le rapport entre la longueur et le diamètre de la chambre de pression (12) en forme de canal est de l'ordre de 2 à 10.

3. Instrument selon la revendication 1 ou 2, dans lequel la pièce de percussion (40) est guidée coaxialement à l'élément de transmission (30).

4. Instrument selon l'une des revendications 1 à 3, dans lequel le dispositif de génération d'ondes de pression (4) génère les ondes de pression périodiquement.

5. Instrument selon l'une des revendications 1 à 4, dans lequel la section de la chambre de pression (12) est élargie en cône du côté distal par rapport à la membrane (16).

6. Instrument selon l'une des revendications 1 à 4, dans lequel la section de la chambre de pression (12) s'amincit en cône du côté distal par rapport à la membrane (16).

7. Instrument selon l'une des revendications 1 à 6, dans lequel la membrane (16) est fixée de façon amovible au dispositif de transmission (8).

8. Instrument selon l'une des revendications 1 à 7, dans lequel un dispositif de serrage (20) maintient la membrane (16) en exerçant une précontrainte.

9. Instrument selon l'une des revendications 1 à 8, dans lequel la membrane (16) est constituée d'une feuille de matière plastique adaptée en impédance à la densité d'un fluide.

10. Instrument selon l'une des revendications 1 à 9, dans lequel le liquide (14) contenu dans la chambre de pression (12) est une solution saline.

11. Instrument selon l'une des revendications 1 à 10, dans lequel une substance d'adaptation d'impédance, qui améliore l'injection des ondes de pression dans le tissu biologique, est placée entre la membrane (16) et le tissu biologique.
